# EUROPEAN PATENT SPECIFICATION

(11) **EP 4 180 414 B1**
(45) Date of publication and mention of the grant of the patent: **26.11.2025**
(21) Application number: 21842464.6
(22) Date of filing: 21.06.2021
(51) Int. Cl.: C07C 51/41, C07C 59/265

(54) **METHOD FOR MANUFACTURING MONO CALCIUM CITRATE BY USING SHELL AND APPLICATION THEREOF**
VERFAHREN ZUR HERSTELLUNG VON MONOCALCIUMCITRAT UNTER VERWENDUNG VON SCHALE UND ANWENDUNG DAVON
PROCÉDÉ DE FABRICATION DE CITRATE MONOCALCIQUE PAR UTILISATION DE COQUES ET APPLICATION ASSOCIÉE

(30) Priority: 13.07.2020 KR 20200086269
(43) Date of publication of application: 17.05.2023
(73) Proprietor: Korea Research Institute of Chemical Technology, Daejeon 34114 (KR)
(72) Inventor: PARK, Joung Kyu, Daejeon 34114 (KR); KANG, Myung Hyun, Daejeon 34114 (KR); LIM, Jieun, Daejeon 34114 (KR)
(74) Representative: Gassner, Birgitta
(86) International application number: PCT/KR2021/007724
(87) International publication number: WO 2022/014884

(56) References cited:
- WO-A1-2014/046344
- CN-A- 102 921 036
- CN-A- 102 921 036
- GB-A- 597 936
- JP-A- H06 121 981
- JP-A- H06 121 981
- JP-A- H08 157 380
- KR-A- 20060 012 954
- KR-B1- 101 499 292

## Description

### BACKGROUND

### Technical Field

The present invention relates to a method for manufacturing calcium dihydrogen citrate (C₁₂H₁₄CaO₁₄) by using shell and application thereof.

### Background Art

For the treatment of shells and recycling of resources, efforts have been made to utilize the same as a landfill or as a construction material and fertilizer.

In particular, there have been attempts to obtain calcium oxide (CaO) by heat-treating shells at 900° C or higher to use as a desulfurization material in power plants or steel mills, and reacting it with water to produce liquid slaked lime. As a prior art related to this, Korean Patent Publication No. 10-2008-0015167 and Korean Patent Registration No. 10-1753823 disclose a method of heat treatment at a high temperature of 800 to 1500° C to obtain calcium carbonate and calcium oxide from shells.

However, this treatment method generates a large amount of carbon dioxide (CO₂) gas during the treatment process, which violates efforts to reduce carbon dioxide (CO₂) gas which is the main culprit of current global warming and too much energy is consumed due to heat treatment. This increases the manufacturing cost and making it difficult to put it into practical use.

Therefore, unlike the prior technology, an eco-friendly and effective processing of shells such as oysters and recycling of resources method is required.

Otherwise, industrial wastewater from electronic product manufacturing plants, LCD manufacturing processes, and semiconductor industries, which are the main domestic industries, contains a lot of hydrofluoric acid. As the industry develops, the amount of hydrofluoric acid wastewater increases rapidly, and treatment of low-concentration and high-concentration hydrofluoric acid wastewater has emerged as an urgent problem due to environmental problems. Currently, the most widely used method for treating hydrofluoric acid wastewater is the precipitation method using liquid slaked lime. However, when such liquid slaked lime is used to remove fluorine, the solubility of slaked lime is low, and the reactivity is lowered and the pH is increased. Accordingly, a larger amount of lime must be injected for fluorine removal, and as a result, a large amount of sludge is generated, resulting in additional treatment costs.

Also, wastewater containing a low concentration of hydrofluoric acid has low efficiency problems since the treatment time is long and the reaction efficiency is low, so slaked lime needs to be input 5 times or more compared to the equivalent weight.

Also, in case of fluorine removal using slaked lime, it is difficult to obtain high-quality calcium fluoride (CaF₂) because the precipitate after reaction with fluorine contains a lot of calcium oxide (CaO) and calcium hydroxide (Ca(OH)₂). In particular manufacturing high purity of calcium fluoride (CaF₂) through the above process, it can be used as a raw material for producing hydrogen fluoride, its recyclability may be high.

As a prior art related to this, Korean Patent Publication No. 10-2016-0090657 discloses a method for removing fluorine using various calcium salts such as calcium carbonate, calcium hydroxide, and calcium nitrate, but the residual fluorine concentration is 12 mg/L and the fluorine removal rate is only about 98.66%, so the residual fluorine concentration cannot be removed up to a low concentration of 5 mg/L. In addition, Korean Patent Registration No. 10-1958079 discloses a method for removing fluorine ions and cyanide using an iron compound and a rare metal, but the fluorine removal rate is only about 91%, so the fluorine removal rate is not high. This is related to the solubility of calcium salts used to remove fluorine, but the solubility of most calcium salts is not so high. Besides, calcium-related materials for use as food additives and health supplements must be dissolved and ionized into calcium ions in order to sufficiently exhibit their effects, and must not be suspended and precipitated, and must have a high dissolution rate and a high degree of ionization in order to increase the absorption rate of calcium in the body. As a prior art related to this invention, GB597936A describes preparing mono-calcium citratedihydrate, Ca(C₆H₇O₇)₂·2H2O. This method comprises mixing calcium carbonate, citric acid and water, followed by adding acetone to remove water or excess citric acid.

JPH06121981A discloses the treatment of sucrose-fluorine waste, using calcium compounds.

Accordingly, in the present invention, manufacturing method has been developed by dissolving shells with citric acid to treated the shells being eco-friendly and increase the solubility. And the present invention was completed by developing a method for recycling shells, in which calcium dihydrogen citrate (C₁₂H₁₄CaO₁₄) prepared by the above manufacturing method is used as a fluorine remover for removing fluoride ions in industrial wastewater or as a food additive replacing conventional calcium citrate.

### DETAILED DESCRIPTION OF THE INVENTION

### Technical Problem

An object of the present invention is to provide a method for manufacturing calcium dihydrogen citrate Ca(C₆H₇O₇)₂by using shell and applications thereof for the treatment of shells and recycling of resources.

### Technical Solution

In order to achieve the above purpose, the present invention provides a method for manufacturing calcium dihydrogen citrate by using shell comprising:
1) a step of preparing a citric acid aqueous solution by dissolving citric acid in water;
2) a step of preparing liquid calcium dihydrogen citrate (C₁₂H₁₄CaO₁₄) by adding shell powder to the citric acid aqueous solution and then stirring:
3) a step of heat drying or quick drying the liquid calcium dihydrogen citrate;
4) a step of recovering calcium dihydrogen citrate (C₁₂H₁₄CaO₁₄) Powder;
The heat drying is performed in an oven at 50° C to 80° C, and the rapid drying is performed by freeze drying or spray drying.

In step 2), the shell powder and citric acid are mixed in a molar ratio of 1:0.8 to 1:5, and the stirring is performed for 1 minute to 300 minutes. After step 2), further step of filtering the liquid calcium dihydrogen citrate to remove impurities may be included.

A composition described herein for removing fluorine comprises calcium dihydrogen citrate prepared by the manufacturing method, wherein the composition includes food, animal feed, plant fertilizer or cosmetic form.

A composition described herein for treating wastewater comprises the calcium dihydrogen citrate prepared by the manufacturing method.

A composition described herein for removing fluorine comprises liquid calcium dihydrogen citrate including shell powder and citric acid.

A composition described herein for treating wastewater comprises liquid calcium dihydrogen citrate including shell powder and citric acid.

Also, in another aspect, the present invention provides a method for removing fluoride ions, comprising:
1) a step of preparing a mixed solution in which shell powder and citric acid are dissolved; and
2) a step of removing fluorine ions by adding the mixed solution to a wastewater solution containing fluoride ions.

Also, in another aspect, the present invention provides a method for manufacturing calcium fluoride comprising:
1) a step of preparing a mixed solution in which shell powder and citric acid are dissolved;
2) a step of removing fluorine ions by adding the mixed solution to a wastewater solution containing fluoride ions; and
3) a step of obtaining calcium fluoride (CaF₂) by filtering and drying the reaction precipitate;

### Effects of the invention

The present invention does not use highly toxic and corrosive hydrochloric acid, nitric acid, sulfuric acid, etc., which have been used to treat shells in the past, and by using citric acid harmless to the human body, so that shells can be treated in a way that is harmless to humans as well as marine life, and it has the advantage of being eco-friendly as it can minimize the amount of carbon dioxide produced.

Furthermore, the present invention uses liquid calcium dihydrogen citrate (C₁₂H₁₄CaO₁₄) manufactured by using shell as a fluorine remover to remove fluorine contained in industrial wastewater, therefore the residual concentration of fluorine can be lowered to 15 mg/L or less, the removal rate of fluorine can be raised to 99% or more, and high-purity calcium fluoride (CaF₂) of 99 to 100% can be obtained after fluorine removal.

Also, calcium dihydrogen citrate manufactured by using shell according to the present invention can be used as a substitute for fields where conventionally expensive calcium citrate is used, such as food additives, animal feeds and cosmetics, and there is an advantage in that it can be used for resource recycling while processing the remaining shells, which are a big problem in coastal areas such as the West Sea and the South Sea.

### BRIEF DESCRIPTION OF THE DRAWINGS

FIG. 1 is a flowchart showing a method for manufacturing calcium dihydrogen citrate according to an aspect of the present invention.
FIG. 2 is a flowchart showing a method for removing fluoride ions and a method for manufacturing calcium fluoride according to another aspect of the present invention.
FIG. 3 is an X-ray diffraction analysis graph analyzing the precipitate formed by the fluorine removal experiment of Examples 1 to 3 of the present invention.

### DETAILED DESCRIPTION

Hereinafter, preferred embodiments of the present invention will be described with reference to the accompanying drawings. However, the embodiments of the present invention can be modified in various forms, and the scope of the present invention is not limited to the embodiments described below. In addition, the embodiments of the present invention are provided to more completely explain the present invention to those skilled in the relevant technical field. Also, in the entire specification, "comprises" a component means that it may include other elements, not to exclude other elements unless otherwise stated.

The present invention provides a method for manufacturing calcium dihydrogen citrate by using shell comprising:
1) a step of preparing a citric acid aqueous solution by dissolving citric acid in water;
2) a step of preparing liquid calcium dihydrogen citrate (C₁₂H₁₄CaO₁₄) by adding shell powder to the citric acid aqueous solution and stirring;
3) a step of heat drying or rapid drying the liquid calcium dihydrogen citrate; and
4) a step of recovering calcium dihydrogen citrate (C₁₂H₁₄CaO₁₄) powder.

Hereinafter, a method for manufacturing liquid calcium dihydrogen citrate (C₁₂H₁₄CaO₁₄) or calcium dihydrogen citrate (C₁₂H₁₄CaO₁₄) powder using shell according to an aspect of the present invention will be described in detail step by step with reference to the Figures.

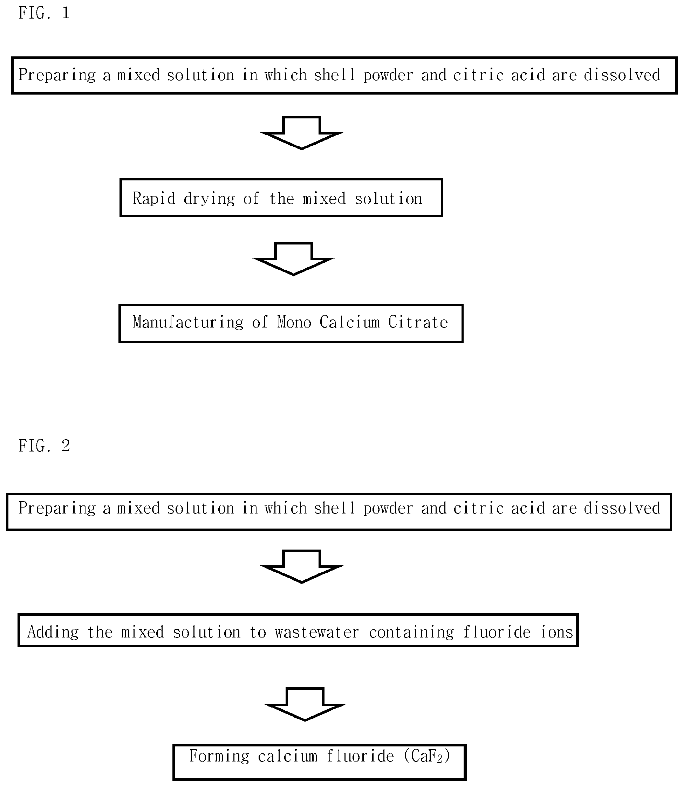

FIG. 1 is a flowchart showing a step-by-step method for manufacturing calcium dihydrogen citrate by using shell according to an aspect of the present invention.

First, liquid calcium dihydrogen citrate (C₁₂H₁₄CaO₁₄) may be prepared by stirring a mixed solution of shell powder and citric acid aqueous solution. The step is a step of dissolving the shell powder by stirring with an aqueous solution of citric acid.

At this time, the shell powder may be prepared by crushing oyster shells, wherein the oyster shells may not be subjected to a heat treatment, and may comprise calcium carbonate (CaCO₃) as a main component.

Also, the shell powder may be prepared by removing impurities attached to the oyster shells, washing them, and then pulverizing them. In this case, the pulverization may be wet pulverization or dry pulverization, and preferably may be pulverization after drying the washed shells.

The pulverized shell powder may have a size of 1 µm to 500 µm.

Citric acid (C₆H₈O₇ or HOC(CO₂H)(CH₂CO₂H)₂) can dissolve shell powder containing calcium carbonate (CaCO₃) as a main component, and in this step, since shells are dissolved using harmless citric acid instead of hydrochloric acid, nitric acid, sulfuric acid, etc., which are highly toxic and corrosive, it is being eco-friendly as the shells are easily treated in a method that is harmless to humans as well as marine life.

In the step 2), at least 0.8 moles of citric acid may be mixed with respect to 1 mole of the shell powder, and preferably, the shell powder and citric acid may be mixed in a molar ratio of 1:0.8 to 1:5. This is to prevent occurrence of precipitation as tricalcium citrate in a later step and to form liquid calcium dihydrogen citrate composed of pure calcium dihydrogen citrate.

Calcium dihydrogen citrate is a compound represented by Formula 1, and can be formed by reacting 2 molecules of citric acid with 1 calcium.

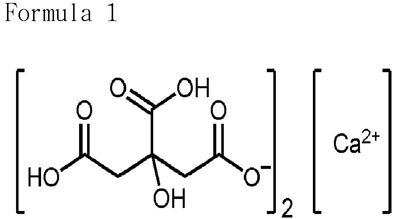

Therefore, when less than 0.8 mole of citric acid is mixed with react to 1 mole of the shell powder, it can be a problem that the amount of citric acid used is small, so that all of the used shells are not dissolved or precipitation may occur during stirring to form tricalcium citrate with low solubility. When citric acid is mixed in an amount exceeding 5 moles relative to 1 mole of the shell powder, excessive use of citric acid that does not participate in the reaction may occur.

The liquid calcium dihydrogen citrate (C₁₂H₁₄CaO₁₄) is a solution in which shell powder and citric acid are dissolved, and may include calcium ions (Ca²⁺) and citrate salt (C₆H₄O₇⁻) dissolved from the shell powder.

Also, calcium dihydrogen citrate powder may be formed from the liquid calcium dihydrogen citrate (C₁₂H₁₄CaO₁₄) by heat drying or rapid drying.

Therefore, stirring for the reaction of the shell powder and citric acid is preferably performed within a time range in which tricalcium citrate does not precipitate out from the liquid calcium dihydrogen citrate (C₁₂H₁₄CaO₁₄), preferably for 1 minute to 300 minutes, more preferably, for 1 minute to 60 minutes, and even more preferably for 1 minute to 20 minutes. If the shell and citric acid react for longer than the stirring time to form a precipitate, crystalline tricalcium citrate may be formed and calcium dihydrogen citrate may not be formed.

At this time, the stirring may be performed using a bar or an ultrasonic stirrer, but it' s not limited, and other suitable stirring methods may be used.

Also, in step 2), high-temperature heat treatment is not required, shells can be dissolved at room temperature of 10° C to 40° C, and carbon dioxide is not generated during dissolution, which is more environmentally friendly than conventional shell treatment methods that require high-temperature heat treatment of 800° C or higher or generate carbon dioxide.

Also, the method for manufacturing calcium dihydrogen citrate by using the shell of the present invention may include a step of heat drying or rapid drying the liquid calcium dihydrogen citrate (C₁₂H₁₄CaO₁₄).

At this time, the heat drying or rapid drying may be performed to manufactured calcium dihydrogen citrate by using shells, but to prepare calcium dihydrogen citrate having a higher supply rate of calcium ions due to high solubility in aqueous solution.

The heat drying is performed in a range of at 50° C to 80° C, and the rapid drying is performed by mixing and stirring the shells and citric acid at normal pressure or room temperature to form calcium dihydrogen citrate by reducing pressure or reducing temperature before tricalcium citrate is formed, and it can be performed by lyophilization or spray drying.

Also, the method for manufacturing calcium dihydrogen citrate by using shell of the present invention may further comprise, after step 2), step of filtering the liquid calcium dihydrogen citrate to remove impurities may be included.

The removing of the impurities is a step of removing residual organic materials, and the organic materials may be separated and removed from the liquid calcium dihydrogen citrate through a filtration process.

The recovered calcium dihydrogen citrate powder may have an average particle size of 100 nm to 10 µm.

The prepared calcium dihydrogen citrate (C₁₂H₁₄CaO₁₄) has a solubility 8 times higher than that of tri-calcium citrate (C₁₂H₁₀Ca₃O₁₄), which is a compound represented by Formula 2.

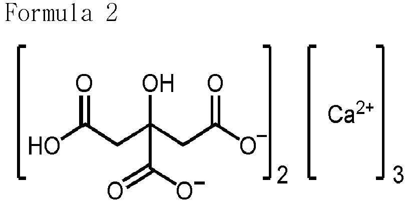

A composition described herein for removing fluorine comprises calcium dihydrogen citrate prepared by the above manufacturing method. A composition described herein for treating wastewater comprises calcium dihydrogen citrate prepared by the above manufacturing method.

The calcium dihydrogen citrate (C₁₂H₁₄CaO₁₄) has significantly higher solubility in a fluorine-containing solution or waste water than calcium carbonate, calcium hydroxide, or tricalcium citrate, and can provide more calcium ions (Ca²⁺). It means the removal effect of fluoride ions in a fluorine-containing solution or waste water may depend on the solubility of calcium contained in the fluorine scavenger, and in the case of slaked lime or quicklime, which is conventionally used as a general fluorine removal agent, the solubility is significantly low, and it is difficult to lower the residual concentration of fluorine to 15 mg/L or less, for this purpose, it is difficult to treat the remaining sludge due to the use of an excessive amount of slaked lime or quicklime, whereas the composition for removing fluorine or the composition for treating wastewater of the present invention can provide a large amount of calcium ions (Ca²⁺) in a solution or waste water containing fluorine, thereby increasing the efficiency of removing fluorine and forming calcium fluoride (CaF₂).

The composition for removing fluorine or the composition for treating wastewater can lower the concentration of fluoride ions in wastewater to 15 mg/L or less, which is the upper limit allowed to be discharged into the sea, and to 3 mg/L or less, which is the upper limit allowed to be discharged into rivers. For example, when fluoride ions are added to a solution at a concentration of 1000 ppm, residual fluorine can be lowered to 3 ppm or less.

Also, the composition for removing fluorine or the composition for treating wastewater can remove fluorine ions with a removal rate of 99.8% or more and obtain calcium fluoride (CaF₂) at the same time, and the calcium fluoride (CaF₂) produced in this way can be reused to produce hydrogen fluoride.

Accordingly, the composition for removing fluorine or the composition for treating wastewater is being eco-friendly that manufactured without high temp heat treatment or strong acid treatment of the shells and is manufactured by recycling shells classified as industrial waste.

Also, the composition for removing fluorine may include food, animal feed, plant fertilizer, or cosmetics.

The calcium dihydrogen citrate has high solubility in the solution and can provide a large amount of calcium ions, stabilizing calcium ions and increasing the absorption rate of calcium in the body, and can be added to food, beverages, cosmetics, etc. for the purpose of calcium reinforcement. Accordingly, the composition comprising calcium dihydrogen citrate can be variously used in the field of functional beverages containing calcium ions, beverages such as functional mineral water, of food processing such as calcium ionized tofu, ice cream, and kimchi, of grain processing such as functional rice and functional flour, of fertilizers such as meat quality improvers, nutrients for vegetables, of animal feeds, of health supplements, of and cosmetics.

Thus, the composition containing the calcium dihydrogen citrate strongly inhibits the production of substances that cause sagging and sagging skin, makes the skin more taut and healthy, and may be effective in promoting the synthesis of the lipid layer, which reinforces the skin's protective film, and may be helpful in preventing troubles caused by calcium deficiency. Moreover, by promoting calcium absorption, calcium concentration in the body can be increased, thereby helping to prevent various adult diseases such as osteoporosis, rickets, skeletal diseases such as tetani, circulatory system diseases, colon diseases, and high blood pressure.

A composition described herein for removing fluorine comprises liquid calcium dihydrogen citrate comprising shell powder and citric acid.

A composition described herein for treating wastewater comprises liquid calcium dihydrogen citrate comprising shell powder and citric acid.

The composition for removing fluorine and the composition for wastewater treatment comprising liquid calcium dihydrogen citrate comprising the shell powder and citric acid can treat fluoride ions in the wastewater by reacting calcium ions (Ca²⁺) with fluoride ions (F⁻) contained in the wastewater thereby precipitating calcium fluoride (CaF₂).

In another aspect, the present invention provides a method for removing fluoride ions, comprising:
1) a step of preparing a mixed solution in which shell powder and citric acid are dissolved; and
2) a step of removing fluorine ions by adding the mixed solution to a wastewater solution containing fluoride ions.

In another aspect, the present invention provides a method for manufacturing calcium fluoride comprising:
1) a step of preparing a mixed solution in which shell powder and citric acid are dissolved;
2) a step of removing fluorine ions by adding the mixed solution to a wastewater solution containing fluoride ions;
3) a step of obtaining calcium fluoride (CaF₂) by filtering and drying the reaction precipitate.

Hereinafter, a method for removing fluoride ions in wastewater and a method for manufacturing calcium fluoride by using shells according to an aspect of the present invention will be described in detail with reference to the drawings.

FIG. 2 is a flowchart showing a method for removing fluoride ions and a method for manufacturing calcium fluoride step by step according to another aspect of the present invention.

The method for removing fluoride ions according to one aspect of the present invention has the advantage of increasing the treatment and recycling of shells.

In the method for removing fluoride ions according to one aspect of the present invention, the mixed solution is a solution in which stirring is stopped before tricalcium citrate having low solubility is precipitated. The mixed solution in which the shell powder and citric acid are dissolved may comprise at least 0.8 mol or more of the citric acid relative to 1 mol of the shell powder, and preferably comprise the shell powder and citric acid in a molar ratio of 1:0.8 to 1:5.

This is to form calcium dihydrogen citrate in a later step, when the citric acid is mixed in an amount of less than 0.8 moles of citric acid relative to 1 mole of the shell powder, the amount of citric acid used is small, so that all of the shells used may not be dissolved or precipitate as tricalcium citrate, and when citric acid is mixed in an amount exceeding 5 moles relative to 1 mole of the shell powder, excessive use of citric acid that does not participate in the reaction may occur.

The mixed solution in which the shell powder and citric acid are dissolved may be formed by adding the shell powder to a citric acid aqueous solution and stirring it.

Therefore, in the process of dissolving the shells, high-temperature heat treatment is not required and carbon dioxide is not generated, which is more environmentally friendly than conventional shell treatment methods that require high-temperature heat treatment of 100° C or higher or generate carbon dioxide.

Calcium ions (Ca²⁺) contained in the mixed solution react with fluoride ions (F⁻) contained in the wastewater to precipitate calcium fluoride (CaF₂), thereby treating fluoride ions in the wastewater solution.

The fluoride ion removal method of the present invention can remove fluoride ions in wastewater from shells and also obtain high-purity calcium fluoride (CaF₂), and the obtained calcium fluoride (CaF₂) is reused for manufacturing hydrogen fluoride (HF), and thus has the advantage of remarkably increasing the degree of recycling of shells.

Hereinafter, examples of the present invention will be described in detail, but the present invention is not limited to the following examples.

### Example 1 - Preparation of calcium dihydrogen citrate

The shells were pulverized by using a grinder, and 1,000 g of the pulverized shell powder was placed in a container containing 10 L of distilled water and 3,843 g of citric acid, and stirred by using a stirrer for 5 to 20 minutes to dissolve the shells.

The solution in which the shells were dissolved (mol ratio of shells and citric acid = 1:2) was filtered, and the filtered solution was dried to recover calcium dihydrogen citrate powder.

### Example 2 - Preparation of tricalcium citrate

The shells were pulverized by using a grinder, and 1,000 g of the pulverized shell powder was put into a container containing 10 L of distilled water and 980 g of citric acid, and stirred by using a stirrer for a time range of 25 to 60 minutes to dissolve the shells. The solution (mol ratio of shell and citric acid = 2:1) turned cloudy immediately after dissolution progressed over time, and precipitates were generated. The solution containing the precipitate was filtered to separate organic substances and residues, and the cloudy solution was dried to recover tricalcium citrate powder.

### Example 3 - Preparation of mixed solution of shell and citric acid

The shells were pulverized by using a grinder, and 8.3 g of the pulverized shell powder was put in a container containing 1 L of distilled water and 8.3 g of citric acid, and stirred for about 10 minutes by using a stirrer to completely dissolve the shells.

As the solution (mol ratio of shell and citric acid = 2:1) was stirred, it was observed that the solution turned slightly yellow over time, which means that as the shell dissolved, organic matter and debris attached to the shell remained. The solution was filtered to separate and remove the solution, organic matter and residue.

### Experimental Example 1 - Analysis of structure and organic matter of calcium dihydrogen citrate

In order to analyze the structure and organic matter of the calcium dihydrogen citrate prepared in Examples 1 and 2. it was analyzed by using an ICP-AES measuring instrument and an elemental analyzer, and the results are shown in Table 1.

At this time, the ICP-AES measuring instrument used the iCAP 6500 duo Inductively Coupled Plasma-Emission Spectrometer model from Thermo Scientific, and as an elemental analyzer, a FLASH EA-2000 Organic Elemental Analyzer from Thermo Scientific was used.

**Table 1**

| | Element content (%) of Example 1 | Theoretical content (%) of calcium dihydrogen citrate (C₁₂H₁₄CaO₁₄) structure | Element content (%) of Example 2 | Theoretical content (%) of tricalcium citrate (C₁₂H₁₀Ca₃O₁₄) structure |
|---|---|---|---|---|
| Ca | 8.37 | 8.41 | 21.1 | 21.08 |
| C | 30.28 | 30.26 | 25.88 | 25.26 |
| H | 3.81 | 4.23 | 2.70 | 3.18 |
| N | 0.055 | - | 0.11 | - |

As shown in Table 1, it can be seen that the powder recovered in Example 1 is consistent with calcium dihydrogen citrate (C₁₂H₁₄CaO₁₄), and it can be seen that the powder recovered in Example 2 is consistent with tricalcium citrate (C₁₂H₁₀Ca₃O₁₄). From this, it can be seen that calcium dihydrogen citrate (C₁₂H₁₄CaO₁₄) can be prepared by reacting the shell with citric acid and heat drying or rapid drying before precipitation occurs through the reaction.

### Experimental Example 2 - Measurement of residual fluorine concentration after fluorine removal experiment

In order to confirm the fluorine removal rate using the powder or solution prepared in Examples 1 to 3, a fluorine removal experiment was performed by the following method, and the residual fluorine concentration of the filtrate remaining after the experiment was measured using an ion concentration meter and is shown in Tables 2 to 4. At this time, a Metrohm 930 compact IC Flex model from Metrohm was used as the ion concentration meter.

### Fluorine removal experiment by using calcium dihydrogen citrate prepared from the shell of Example 1

A 50% hydrofluoric acid (HF) solution with a concentration of 1,000 ppm was prepared, and so that the molar ratio of calcium ion:fluoride ion is 1:2, 2:2, 10.56 g and 21.12 g of the calcium dihydrogen citrate powder recovered in Example 1 were mixed with 1 L of water to make a solution, the hydrofluoric acid and the solution were reacted for 30 minutes, and the results are shown in Table 2.

**Table 2**

| | molar ratio of Calcium ion : Fluoride ion | Amount of calcium dihydrogen citrate added (g) | Residual fluorine concentration (ppm) | removal rate (%) |
|---|---|---|---|---|
| Control group | | - | 970 | - |
| Example 1 | 1:2 | 10.56 | 7.3 | 99.25 |
| Example 1 | 2:2 | 21.12 | 2.2 | 99.77 |

As shown in Table 2, in Fluorine Removal Test Results when the calcium dihydrogen citrate powder of Example 1 was added at a Ca:F molar ratio of 1:2 and 2:2, in a solution containing fluoride ions at a concentration of 1,000 ppm each (experimental value: 970 ppm), it can be seen that the residual fluorine concentrations were 7.3 ppm and 2.2 ppm, the fluorine removal rates were 99.25% and 99.77%, the residual fluorine concentration was 15 ppm or less, and the fluorine removal rate was 99% or more, indicating a very excellent fluorine removal effect.

### Fluorine removal experiment with tricalcium citrate prepared from shells of Example 2

A 50% hydrofluoric acid (HF) solution with a concentration of 1,000 ppm was prepared, 4.75 g, 9.5 g, and 19.1 g of the tricalcium citrate powder recovered in Example 2 were mixed with 1 L of water so that the molar ratio of calcium ion: fluoride ion was 1:2, 2:2, and 4:2 to form a slurry, the hydrofluoric acid and the slurry were reacted for 30 minutes, and the results are shown in Table 3. (Tricalcium citrate (C₁₂H₁₀Ca₃O₁₄) contains 3 moles of calcium ions per mole, so 1/3 of the theoretical amount is added.)

**Table 3**

| | molar ratio of Calcium ion : Fluoride ion | Amount of tricalcium citrate added (g) | Residual fluorine concentration (ppm) | removal rate (%) |
|---|---|---|---|---|
| Control group | | - | 970 | - |
| Example 2 | 1:2 | 4.75 | 23 | 97.63 |
| Example 2 | 2:2 | 9.5 | 16.2 | 98.33 |
| Example 2 | 4:2 | 19.1 | 16.5 | 98.29 |

As shown in Table 3, in Fluorine Removal Test Results when the calcium dihydrogen citrate powder of Example 2 was added at a Ca:F molar ratio of 1:2, 2:2 and 4:2, in a solution containing fluoride ions at a concentration of 1,000 ppm each (experimental value: 970 ppm), it can be seen that the residual fluorine concentrations were 23 ppm, 16.2 ppm and 16.5 ppm, the fluorine removal rates were 97.63%, 98.33% and 98.28%. Although the fluorine removal effect is excellent, the effect is somewhat inferior to that of the calcium dihydrogen citrate of Example 1. Also, it can be confirmed that even when the tricalcium citrate powder of Example 2 is added twice as much as the theoretical equivalent, the fluorine removal rate does not increase and reaches the limit.

From this, it can be seen that calcium dihydrogen citrate manufactured by using shell by the manufacturing method of the present invention can lower the residual concentration of fluorine to 10 ppm or less and increase the fluorine removal rate to 99% or more. This may be significantly superior to conventional calcium salts for removing fluorine, such as tricalcium citrate, slaked lime, quicklime, liquid slaked lime, calcium carbonate and calcium hydroxide.

### Fluorine removal experiment using the mixed solution in which shell and citric acid were dissolved in Example 3

A 50% hydrofluoric acid (HF) solution with a concentration of 1,000 ppm was prepared, the solution in which the shells and citric acid were dissolved prepared in Example 3 was mixed with 1 L of water and reacted for 30 minutes, and the results are shown in Table 4.

**Table 4**

| | amount of shell added (g) | Residual fluorine concentration (ppm) | removal rate (%) |
|---|---|---|---|
| Control Group | - | 970 | - |
| Example 3 | 8.3g | 1.9 | 99.81 |

As shown in Table 4, when the solution in which the shell and citric acid were dissolved prepared in Example 3 was added to a solution containing fluoride ions at a concentration of 1,000 ppm, it can be seen that the residual fluorine concentration is very low as 1.9 ppm and the fluorine removal rate is remarkably high as 99.81%. This can be attributed to the fact that when the shell is dissolved using citric acid, a large amount of calcium ions are dissolved and come out. From this, it can be seen that the solution in which shells and citric acid are dissolved can be effectively used to remove fluoride ions in a solution containing fluoride ions.

### Example 3 - X-ray diffraction analysis of precipitate after fluorine removal experiment

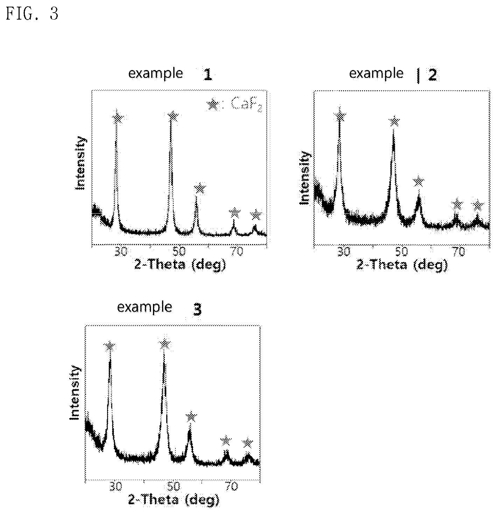

In order to analyze the components of the precipitate produced after the fluorine removal experiment of Experimental Example 2, in Example 2, X-ray diffraction analysis (XRD) was performed on the precipitate filtered and dried through the fluorine removal experiment of Examples 1 to 3, and the results are shown in FIG. 3. In the experiment, the X-ray diffractometer was analyzed using Rigaku's D/MAX2200V/PC model.

As shown in FIG. 3, as a result of component analysis of the precipitate produced after the fluorine removal experiments in Examples 1 to 3, the precipitate is calcium fluoride (CaF₂), and no other impurities are detected. From this, it can be seen that the precipitate formed by reacting a solution of shell and citric acid (Example 3) or calcium dihydrogen citrate prepared therefrom (Examples 1 and 2) with a solution containing fluorine ions is calcium fluoride (CaF₂), wherein the precipitate contains almost no impurities and unreacted substances.

Accordingly, it can be seen that the fluorine removal method using the shell of the present invention can effectively remove fluorine ions from a solution containing fluoride ions and recover high-purity calcium fluoride (CaF₂).

## Claims

1. A method for manufacturing calcium dihydrogen citrate using shell comprising:
1) a step of preparing a citric acid aqueous solution by dissolving citric acid in water;
2) a step of manufacturing liquid calcium dihydrogen citrate (C₁₂H₁₄CaO₁₄) by adding shell powder to the citric acid aqueous solution and stirring;
3) a step of heat drying or rapid drying the liquid calcium dihydrogen citrate; and
4) a step of recovering calcium dihydrogen citrate (C₁₂H₁₄CaO₁₄) powder.

2. The method for manufacturing calcium dihydrogen citrate by using shell according to claim 1, wherein the heat drying is performed in an oven at 50° C to 80° C, wherein the rapid drying is performed by lyophilization or spray drying.

3. The method for manufacturing calcium dihydrogen citrate by using shell according to claim 1, wherein in step 2), the shell powder and citric acid are mixed in a molar ratio of 1:0.8 to 1:5.

4. The method for manufacturing calcium dihydrogen citrate by using shell according to claim 1, wherein the stirring is performed for 1 minute to 300 minutes.

5. The method for manufacturing calcium dihydrogen citrate by using shell according to claim 1, further comprising a step of filtering the liquid calcium dihydrogen citrate to remove impurities, after the step 2).

6. A method for removing fluoride ions, comprising:
1) a step of preparing a mixed solution in which shell powder and citric acid are dissolved;
2) a step of removing fluorine ions by adding the mixed solution to a wastewater solution containing fluoride ions.

7. A method for manufacturing calcium fluoride comprising:
1) a step of preparing a mixed solution in which shell powder and citric acid are dissolved;
2) a step of removing fluorine ions by adding the mixed solution to a wastewater solution containing fluoride ions;
3) a step of obtaining calcium fluoride (CaF₂) by filtering and drying the reaction precipitate.

## Patentansprüche

1. Verfahren zur Herstellung von Calciumdihydrogencitrat unter Verwendung von Schalen, umfassend:
1) einen Schritt zur Herstellung einer wässrigen Zitronensäurelösung durch Auflösen von Zitronensäure in Wasser;
2) einen Schritt zur Herstellung von flüssigem Calciumdihydrogencitrat (C₁₂H₁₄CaO₁₄) durch Zugeben von Schalenpulver zu der wässrigen Zitronensäurelösung und Rühren;
3) einen Schritt der Wärmetrocknung oder Schnelltrocknung des flüssigen Calciumdihydrogencitrats; und
4) einen Schritt der Gewinnung von Calciumdihydrogencitrat (C₁₂H₁₄CaO₁₄) in Form eines Pulvers.

2. Verfahren zur Herstellung von Calciumdihydrogencitrat unter Verwendung von Schalen nach Anspruch 1, wobei die Wärmetrocknung in einem Ofen bei 50 °C bis 80 °C durchgeführt wird, wobei die Schnelltrocknung durch Lyophilisierung oder Sprühtrocknung durchgeführt wird.

3. Verfahren zur Herstellung von Calciumdihydrogencitrat unter Verwendung von Schalen nach Anspruch 1, wobei in Schritt 2) das Schalenpulver und die Zitronensäure in einem Molverhältnis von 1 : 0,8 bis 1 : 5 gemischt werden.

4. Verfahren zur Herstellung von Calciumdihydrogencitrat unter Verwendung von Schalen nach Anspruch 1, wobei das Rühren für 1 Minute bis 300 Minuten durchgeführt wird.

5. Verfahren zur Herstellung von Calciumdihydrogencitrat unter Verwendung von Schalen nach Anspruch 1, ferner umfassend nach dem Schritt 2) einen Schritt zur Filterung des flüssigen Calciumdihydrogencitrats, um Verunreinigungen zu entfernen.

6. Verfahren zur Entfernung von Fluoridionen, umfassend:
1) einen Schritt der Herstellung einer gemischten Lösung, in welcher Schalenpulver und Zitronensäure gelöst sind;
2) einen Schritt der Entfernung von Fluorionen durch Zugeben der gemischten Lösung zu einer Abwasserlösung, die Fluoridionen enthält.

7. Verfahren zur Herstellung von Calciumfluorid, umfassend:
1) einen Schritt der Herstellung einer gemischten Lösung, in welcher Schalenpulver und Zitronensäure gelöst sind;
2) einen Schritt der Entfernung von Fluorionen durch Zugeben der gemischten Lösung zu einer Abwasserlösung, die Fluoridionen enthält,
3) einen Schritt der Gewinnung von Calciumfluorid (CaF₂) durch Filterung und Trocknung des Reaktionsniederschlags.

## Revendications

1. Procédé de fabrication de dihydrogénocitrate de calcium à l'aide de coquilles comprenant :
1) une étape de préparation d'une solution aqueuse d'acide citrique par dissolution d'acide citrique dans l'eau ;
2) une étape de fabrication de dihydrogénocitrate de calcium (C₁₂H₁₄CaO₁₄) liquide par ajout d'une poudre de coquilles à la solution aqueuse d'acide citrique et mélange ;
3) une étape de séchage par la chaleur ou de séchage rapide du dihydrogénocitrate de calcium liquide ; et
4) une étape de récupération de la poudre de dihydrogénocitrate de calcium (C₁₂H₁₄CaO₁₄).

2. Procédé de fabrication de dihydrogénocitrate de calcium à l'aide de coquilles selon la revendication 1, dans lequel le séchage par la chaleur est effectué dans un four à une température de 50°C à 80°C, dans lequel le séchage rapide est effectué par lyophilisation ou séchage par pulvérisation.

3. Procédé de fabrication de dihydrogénocitrate de calcium à l'aide de coquilles selon la revendication 1, dans lequel, dans l'étape 2), la poudre de coquilles et l'acide citrique sont mélangés en un rapport molaire de 1:0,8 à 1:5.

4. Procédé de fabrication de dihydrogénocitrate de calcium à l'aide de coquilles selon la revendication 1, dans lequel le mélange est effectué pendant une minute à 300 minutes.

5. Procédé de fabrication de dihydrogénocitrate de calcium à l'aide de coquilles selon la revendication 1, comprenant en outre une étape de filtration du dihydrogénocitrate de calcium liquide pour enlever les impuretés, après l'étape 2).

6. Procédé de suppression des ions fluorure, comprenant :
1) une étape de préparation d'une solution mixte dans laquelle de la poudre de coquilles et de l'acide citrique sont dissous ;
2) une étape de suppression des ions fluor par ajout de la solution mixte à une solution d'eau usée contenant des ions fluorure.

7. Procédé de fabrication de fluorure de calcium comprenant :
1) une étape de préparation d'une solution mixte dans laquelle de la poudre de coquilles et de l'acide citrique sont dissous ;
2) une étape de suppression des ions fluor par ajout de la solution mixte à une solution d'eau usée contenant des ions fluorure ;
3) une étape d'obtention de fluorure de calcium (CaF₂) par filtration et séchage du précipité réactionnel.
